(19) **Europäisches Patentamt / European Patent Office / Office européen des brevets**

(11) **EP 1 317 903 B1**

(12) **FASCICULE DE BREVET EUROPEEN**

(45) Date de publication et mention
de la délivrance du brevet:
**25.07.2007 Bulletin 2007/30**

(51) Int Cl.:
*A61B 6/00* (2006.01)  *A61B 6/03* (2006.01)

(21) Numéro de dépôt: **02292988.9**

(22) Date de dépôt: **04.12.2002**

(54) **Procédé de radiographie à double énergie en distinguant tissus osseux, tissus maigres et tissus gras**

Röntgenverfahren mittels zweier Röntgenstrahlenergien zur Unterscheidung zwischen Knochengewebe, Magergewebe und Fettgewebe

Method of dual-energy radiography for distinguishing bone tissue, lean tissue and fat tissue

(84) Etats contractants désignés:
**AT BE BG CH CY CZ DE DK EE ES FI FR GB GR IE IT LI LU MC NL PT SE SI SK TR**

(30) Priorité: **07.12.2001 FR 0115849**

(43) Date de publication de la demande:
**11.06.2003 Bulletin 2003/24**

(73) Titulaire: **COMMISSARIAT A L'ENERGIE ATOMIQUE**
**75015 Paris (FR)**

(72) Inventeurs:
• **Herve, Lionel**
**38000 Grenoble (FR)**
• **Robert-Coutant, Christine**
**38410 Saint-Martin d'Uriage (FR)**
• **Dinten, Jean-Marc**
**69008 Lyon (FR)**

(74) Mandataire: **Ilgart, Jean-Christophe et al**
**c/o Brevatome,**
**3, rue du Docteur Lancereaux**
**75008 Paris (FR)**

(56) Documents cités:
WO-A-00/21441   US-A- 5 465 284
US-A- 5 917 877   US-B1- 6 173 034
US-B1- 6 205 348   US-B1- 6 282 258

**EP 1 317 903 B1**

**Description**

Cette invention concerne un procédé de radiographie par des rayons X ou γ à double énergie et comprend une distinction entre tissus osseux, tissus maigres et tissus gras, pour des mesures de contenu minéral osseux d'un organisme (ostéodensitométrie) avant tout.

Il est bien connu d'employer des rayons de deux énergies différentes pour irradier un organisme et de mesurer leurs deux atténuations par un réseau surfacique de détecteurs situé derrière l'organisme. En effet, les rapports d'atténuation des tissus osseux et des tissus mous ne sont pas les mêmes pour les deux énergies, si bien qu'on peut calculer les épaisseurs de tissus osseux et de tissus mous traversées par des rayons, le problème numérique revenant à inverser un système de deux équations à deux inconnues pour les mesures de chacun des détecteurs atteints par les rayons qui ont traversé l'organisme.

Les résultats fournis par ce genre de méthode ne peuvent cependant qu'être imprécis puisque les tissus des organismes vivants renferment aussi des graisses, dont les coefficients d'absorption sont différents de ceux des tissus osseux et même des tissus maigres.

Plusieurs tentatives ont été faites pour remédier à cette insuffisance du procédé de base. Il vient à l'esprit qu'une troisième irradiation menée avec des rayons d'une troisième énergie permettrait d'obtenir un système de trois équations dont la solution donnerait les trois épaisseurs traversées des tissus osseux, maigres et gras, mais il est apparu qu'une précision suffisante ne pouvait pas être atteinte en pratique en raison du bruit produit par les photons du rayonnement et de la nécessité de limiter la dose totale d'irradiation reçue par l'organisme. L'article "Theoretical and experimental limits of triple photon energy absorptiometry in the measurement of bone mineral" par Kotzki et d'autres dans la revue Phys. Med. Biol volume 36, 1991, pages 429 à 437, donne des explications plus détaillées.

Dans un autre procédé, appelé "méthode de la ligne de base", on fait l'hypothèse que la proportion de graisse est la même dans les tissus osseux et les tissus mous, mais cela n'est pas toujours vérifié et le calcul peut alors subir des erreurs assez importantes pouvant atteindre 20% de l'épaisseur des tissus osseux. L'article "Effect of nonmineral tissues on measurement of bone mineral content by dual-photon absorptionmetry" par Sorenson et d'autres, Med. Phys. volume 17 (5), 1990, pages 905 à 912, est une illustration de cette méthode.

Enfin, le renseignement supplémentaire nécessaire pour distinguer les trois éléments des organismes peut consister en l'épaisseur totale traversée par les rayons. L'article "Dual-photon absorptiometry for determination of bone mineral content in the calcaneus with correction of fat" par Jonson et d'autres, Phys. Med. Biol., volume 35, numéro 7, 1990, pages 961 à 969, en est une illustration.

Une source d'erreur apparaît toutefois quand la portion étudiée de l'organisme contient des poches gazeuses, car l'épaisseur totale traversée n'est plus la somme des épaisseurs des tissus osseux, maigres et gras et la méthode est mise en échec. L'invention a pour objet de la perfectionner et de permettre notamment d'obtenir et d'employer le total réel de l'épaisseur des tissus osseux, maigres et gras traversés plutôt que l'épaisseur de l'organisme pour la résolution du système. Ainsi, elle concerne un procédé de radiographie par rayons à double énergie comprenant une distinction entre tissus osseux, tissus maigres et tissus gras d'un organisme traversé par les rayons et des estimations d'épaisseurs des tissus maigres, des tissus osseux et des tissus gras traversés par les rayons, caractérisé en ce qu'il comprend les étapes suivantes :

- sélection de certains des rayons, qui n'ont pas traversé de tissus osseux ;
- calcul des épaisseurs de tissus maigres et de tissus gras traversées par les rayons sélectionnés d'après des atténuations subies aux deux épaisseurs de tissus maigres et de tissus gras ;
- calcul des sommes des épaisseurs totales traversées par les rayons dans l'organisme, qui sont égales aux-dites sommes pour les rayons sélectionnés et sont estimées par des interpolations entre lesdites sommes pour d'autres rayons :
- et calcul des épaisseurs de tissus osseux, des tissus maigres et de tissus gras traversées par les rayons d'après les atténuations subies aux deux énergies et les épaisseurs totales.

Voici de façon plus détaillée comment on procède. L'organisme étudié est soumis à une irradiation de rayons à deux énergies par un faisceau qui peut être conique. Les coefficients d'atténuation des tissus osseux, maigres et gras sont connus pour les deux catégories de rayons. La table des valeurs totales d'atténuation mesurées, qui correspond à une double image bidimensionnelle de l'organisme, est relevée. On en dégage une répartition approximative des tissus osseux, soit par hypothèse, soit par un examen des valeurs totales d'atténuations, qui sont sensiblement plus importantes quand des os ont été traversés par les rayons.

On s'intéresse alors aux endroits dépourvus de tissus osseux. La connaissance des deux atténuations et des coefficients pour les tissus maigres et gras permet d'obtenir facilement les épaisseurs de ces deux tissus respectivement traversées par les rayons. Leur somme est la longueur totale d'atténuation du rayonnement par l'organisme à l'endroit concerné, les poches de gaz étant ignorées.

**2**

Cette longueur totale d'atténuation est ensuite estimée partout ailleurs dans l'organisme et notamment aux endroits contenant des tissus osseux. On peut procéder par des interpolations linéaires ou autres entre les endroits où ses valeurs ont déjà été obtenues.

On dispose alors, pour chacun des détecteurs, de trois renseignements (les deux atténuations et la longueur totale d'atténuation) contre trois inconnues que sont les longueurs traversées des trois groupes de tissus, ou leurs masses traversées. Le système d'équation peut être de la forme donnée ci-dessous :

$$\begin{pmatrix} \mu_{1m} & \mu_{1g} & \mu_{1os} \\ \mu_{2m} & \mu_{2g} & \mu_{2os} \\ 1/\rho_m & 1/\rho_g & 1/\rho_{os} \end{pmatrix} \begin{pmatrix} M_m \\ M_g \\ M_{os} \end{pmatrix} = \begin{pmatrix} X_1 \\ X_2 \\ L \end{pmatrix}$$

où $M_m$, $M_g$ et $M_{os}$ désignent les masses par unité de surface (en g/cm$^2$) de tissus maigres, gras et osseux traversées par le rayonnement ; $X_1$, $X_2$ et $L$ désignent les atténuations du rayonnement (exprimées par le logarithme des rapports d'intensités du rayonnement d'origine $I_o$ et du rayonnement I reçu par le détecteur concerné : $X = \ln (I_o/I)$), et la longueur totale d'atténuation ; $\mu_{1m}$, $\mu_{1g}$, $\mu_{1\,os}$, $\mu_{2m}$, $\mu_g$ et $\mu_{2\,os}$ désignent les coefficients d'atténuation, pour les deux rayonnements, des tissus maigres, gras et osseux en cm/g$^2$, et $\rho_m$, $\rho_g$ et $\rho_{as}$ sont les masses volumiques de ces tissus. L'inversion du système donne les valeurs de $M_m$, $M_g$ et $M_{os}$, qu'on peut corréler aux épaisseurs traversées de tissus maigres, gras et osseux $l_m$, $l_g$ et $l_{os}$ par les formules

$$l_m = M_m/\rho_m , \quad l_g = M_g/\rho_g \quad et \quad l_{os} = M_{os}/\rho_{os} .$$

Par rapport à la méthode classique de la ligne de base, l'application de l'invention a donné de très bons résultats. En particulier, une très bonne précision a été constatée dans les régions essentielles des organismes alors que la méthode de la ligne de base a produit des erreurs d'évaluations aux endroits de zones osseuses et surtout de transition entre les zones osseuses et les tissus mous.

L'exécution du procédé détaillé ci-dessus dépend de ce que la longueur totale traversée L est supposée s'exprimer par une fonction linéaire des mesures $X_1$ et $X_2$. Cette hypothèse n'est pas toujours vérifiée, notamment quand le rayonnement est polychromatique : la longueur L s'exprime alors par une fonction polynominale plus compliquée telle que $L = \alpha X_1 + \beta X_2 + \gamma X^2_1 + \delta X_1 X_2 + \varepsilon X^2_2$.

On prévoit alors de développer le procédé précédent par les étapes supplémentaires que voici. Les coefficients $\alpha$, $\beta$, $\gamma$, $\delta$ et $\varepsilon$ sont évalués par des mesures d'irradiation à travers des cales en nombre suffisant, et qui présentent des épaisseurs différentes de matériaux simulant les tissus maigres et gras, comme l'eau et le plexiglas. La longueur totale d'atténuation L peut ensuite être calculée en tout point de l'objet conformément au procédé principal, directement aux endroits dépourvus d'os et par interpolation ailleurs.

Ensuite, comme les longueurs $l_m$, $l_g$ et $l_{os}$ s'expriment alors par des fonctions non linéaires de $X_1$, $X_2$ et L, les coefficients de ces fonctions sont évalués à nouveau par des mesures d'irradiation à travers des cales, qui sont ici composées d'épaisseurs différentes de trois matériaux, à savoir les précédents et un matériau simulant les tissus osseux comme l'hydroxyapatite. Les longueurs (ou les masses) traversées peuvent alors être obtenues.

**Revendications**

1. Procédé de radiographie par rayons à double énergie comprenant une distinction entre tissus osseux, tissus maigres et tissus gras d'un organisme traversé par les rayons et des estimations d'épaisseurs des tissus maigres, des tissus osseux et des tissus gras traversés par les rayons, **caractérisé en ce qu'**il comprend les étapes suivantes :

   - sélection de certains des rayons, qui n'ont pas traversé de tissus osseux ;
   - calcul des sommes des épaisseurs de tissus maigres et de tissus gras traversées par les rayons sélectionnés d'après des atténuations subies auxdites deux épaisseurs de tissus maigres et de tissus gras ;
   - calcul des longueurs totales traversées par les rayons dans l'organisme, qui sont égales auxdites sommes pour les rayons sélectionnés et sont estimées par des interpolations entre lesdites sommes pour d'autres des rayons :

- et calcul des épaisseurs de tissus osseux, des tissus maigres et de tissus gras traversées par les rayons d'après les atténuations subies aux deux énergies et les longueurs totales.

## Claims

1. A radiography process by means of dual-energy rays comprising a differentiation among bone, tissues, lean tissues and fatty tissues of an organism penetrated by the rays and estimations of thicknesses of the lean tissues, bone tissues and fatty tissues penetrated by the rays, **characterized in that** it comprises the following stages:

- selection of certain rays, which have not penetrated bone tissues;
- calculation of the sums of the thicknesses of lean tissues and fatty tissues penetrated by the selected rays according to the attenuations sustained at the said two thicknesses of lean tissues and fatty tissues;
- calculation of the total lengths penetrated by the rays in the organism, which are equal to the said sums for the selected rays and are estimated by means of interpolations between said sums for other rays;
- and calculation of the thicknesses of bone tissues, lean tissues and fatty tissues penetrated by the rays according to the attenuations sustained at the two energies and the total lengths.

## Patentansprüche

1. Röntgenverfahren mittels zweier Röntgenstrahlenergien zur Unterscheidung zwischen Knochengeweben, Magergeweben und Fettgeweben eines von den Strahlen durchquerten Organismus und zur Schätzung der Dicken der von den Strahlen durchquerten Knochengewebe, Magergewebe und Fettgewebe,
**dadurch gekennzeichnet, dass** es die folgenden Schritte umfasst:

- Selektion von bestimmten Strahlen, die keine Knochengewebe durchquert haben;
- Berechnung der Summen der von den selektierten Strahlen durchquerten Magergewebe- und Fettgewebedicken gemäß den durch die genannten beiden Magergewebe- und Fettgewebedicken verursachten Schwächungen;
- Berechnung der in dem Organismus von den Strahlen durchquerten Gesamtlängen, die für die selektierten Strahlen gleich den genannten Summen sind und für andere Strahlen geschätzt werden durch Interpolationen zwischen den genannten Summen;
- und Berechnung der von den selektierten Strahlen durchquerten Knochengewebe-, Magergewebe- und Fettgewebedicken gemäß den Schwächungen beider Energien und den Gesamtlängen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **KOTZKI.** *Phys. Med. Biol,* 1991, vol. 36, 429-437
- **SORENSON.** *Med. Phys.,* 1990, vol. 17 (5), 905-912
- **JONSON.** *Phys. Med. Biol.,* 1990, vol. 35 (7), 961-969